# EUROPEAN PATENT SPECIFICATION

(11) **EP 0 358 712 B1**
(45) Date of publication and mention of the grant of the patent: **13.04.1994**
(21) Application number: 88904878.1
(22) Date of filing: 11.05.1988
(51) Int. Cl.: B32B 3/00, B32B 27/08

(54) **INK-REINFORCED POLYURETHANE FILMS**
MIT TINTE VERSTÄRKTE POLYURETHANFILME
COUCHES MINCES DE POLYURETHANE RENFORCEES PAR ENCRE

(30) Priority: 12.05.1987 US 48932
(43) Date of publication of application: 21.03.1990
(73) Proprietor: CONMED, INC., Utica, NY 13501 (US)
(72) Inventor: ABRAHAM, William, W., New Hartford, NY 13513 (US); GENTELIA, John, S., Madison, NY 13402 (US)
(74) Representative: Fort, Jacques
(86) International application number: US8801568
(87) International publication number: WO8808787

(56) References cited:
- FR-A- 2 440 588
- FR-A- 2 544 982
- US-A- 3 645 835
- WPI FILE SUPPLIER, accession no. 75-50033w, Derwent Publications Ltd, London, GB; & JP-A-49 133 467

## Description

### FIELD OF THE INVENTION

The present invention relates to ink-coated thin films and more specifically polyurethane films which are useful in holding I.V. needles in place.

### BACKGROUND OF THE INVENTION

Adhesive materials provided for use on patients, such as for retaining I.V. needles in place, must provide protection against infection, while at the same time allowing epidermal water to evaporate. The material used must therefore allow a rate of moisture vapor transmission which is neither too low nor too high. Previous attempts at such adhesives, such as U.S. Patent Re. 31,887, have provided a continuous backing material of unreinforced polyurethane film combined with a continuous adhesive. Such membranes often allow too much moisture to pass through, are much more likely to break apart, and are more difficult to apply than reinforced materials. It is therefore desirable to obtain a reinforced polyurethane backing material which maintains an optimal rate of moisture vapor transmission and provides more body for ease of application. It is also desirable to obtain other thin films which also have superior strength and tear resistance.

It is also known by document US-3 645 835 to reinforce suture strips with a continuous fabric layer.

### SUMMARY OF THE INVENTION

It has been discovered that coating portions of polyurethane or other films with ink provides a product of greater stability and applicability with a moisture vapor transmission rate lower than that of the bare membrane. An ink-coated polyurethane membrane, combined with a suitable adhesive, provides a stronger product which still maintains a sufficient level of moisture transmissability, and is thus a highly desirable material for retaining I.V. needles and the like on the skin.

The ink-coated polyurethane or other membranes of the present invention can also be used in applications not requiring an adhesive. For example, it may be more desirable to wrap an arm or other region with sheets of a non-adhesive thin film material. This film can be reinforced using the method of the present invention yet will not require an adhesive backing.

### BRIEF DESCRIPTION OF THE DRAWINGS

Fig. 1 is a perspective view of the reinforced polyurethane membrane of the present invention.

Fig. 2 is a top view of the membrane of the present invention having an alternative pattern of ink-type material.

### DETAILED PRESCIPTION OF THE PREFERRED EMBODIMENTS

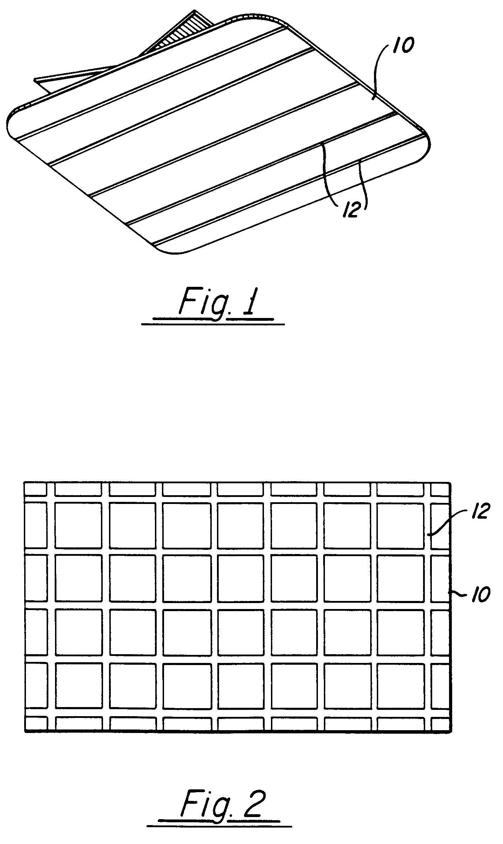

The moisture vapor permeable material of the present invention as observed in Figs. 1 and 2, comprises a polyurethane backing member coated with an ink-type material. The ink-type material 12 is preferably placed on portions of the polyurethane membrane 10 as in the striped configuration observed in Fig. 1. The ink can be printed in any pattern and can be placed on the top or bottom of the membrane 10. Optimally, the ink-type material covers 1 to 85% of the surface area. The pattern indicated in Fig. 1 allows for greater stability and facilitates the removal of the "wing-type" release liner and subsequent application of the product. This and other ink patterns can reinforce the polyurethane membrane enough to eliminate the need for a frame on the product, as is necessary in prior art membranes. Another configuration of ink-type material on the backing member is observed in Fig. 2.

A number of different ink-type coatings have been used successfully to reinforce the polyurethane backing and still maintain sufficient moisture vapor transmission. Ideally, non-toxic inks along with medically approved colorants can be used. One such coating could be a co-solvent polyamide polymer system ink along with copper-phthalocyanine, a federally approved colorant. Another desirable ink material is styrene dissolved in a carrier such as alcohol or methyl ethyl ketone. It is also possible to employ polyethylene as the ink-type material.

The ink-type material can be applied to the polyurethane membrane in any suitable fashion currently known in the art. For instance, the ink can be applied by conventional printing machines and methods. One such method suitable for depositing the ink on the membrane is known as the gravure flex method of printing. Additionally, the pattern of ink can be placed on the polyurethane by applying it in strips of either hot melt material, or of narrow tape. In all of these ways, a pattern of ink-type material can be placed on the polyurethane, resulting in a strengthened, effective product.

When used with a suitable adhesive, the ink-reinforced membranes of the present invention can be used to retain IV needles and the like in place on the skin of a patient. Such adhesive membranes are constructed by placing a suitable adhesive on the underside of the ink-coated polyurethane backing member by any conventional method. The adhesive used should be porous to allow transmission of moisture through the product or can be of the hydrophilic type such as those used with electrodes. The adhesive can be applied continuously or non-continuously across the backing member, forming any desired pattern which allows for sufficient adhesion to the skin. The resulting product is a strengthened moisture vapor-permeable adhesive suitable for use in retaining IV needles, electrodes, or other devices on the skin of the patient.

## Claims

1. A method of reinforcing a moisture-vapor-permeable polyurethane membrane (10) comprising applying a coat of an ink material in a pattern (12) on the polyurethane membrane to strengthen the membrane (10).

2. A method of reinforcing a polyurethane membrane as claimed in claim 1 wherein said material is applied in strips (12).

3. A method of making a reinforced moisture vapor-permeable polyurethane membrane coated with an adhesive comprising coating an adhesive onto the underside of the reinforced membrane according to claim 1.

4. A method as claimed in claim 3 wherein said ink material is applied in strips (12).

5. A film material comprising a continuous moisture vapor permeable member (10) having at least a partial coating of ink developed in a pattern (12) on a surface of said moisture vapor permeable member to provide reinforcement for strengthening said moisture vapor permeable member (10).

6. A film material as claimed in claim 5 wherein said ink comprises a non-toxic ink with a medically approved colorant.

7. A film material as claimed in claim 6, wherein said colorant comprises a copper-phtalocyanine colorant.

8. A film material as claimed in claim 5, wherein said ink comprises styrene dissolved in a carrier or polyethylene.

9. A film material as claimed in claim 8, wherein the carrier comprises methyl ethyl ketone or alcohol.

10. A film material according to claim 5, further including an adhesive coated on said moisture vapor permeable member.

## Patentansprüche

1. Verfahren zum Verstärken einer feuchtigkeit-dampf-permeablen Polyurethan-Membran (10), bei welchem Verfahren eine dünne Schicht eines Tintenmaterials in einem Muster (12) auf der Polyurethan-Membran aufgebracht wird, um die Membran (10) zu versteifen.

2. Verfahren zum Verstärken einer Polyurethan-Membran gemäß Anspruch 1, bei welchem das Material in Streifen (12) aufgebracht wird.

3. Verfahren zum Herstellen einer verstärkten feuchtigkeitdampf-permeablen Polyurethan-Membran, auf die ein Klebemittel aufgebracht ist, bei welchem ein Klebemittel auf die Unterseite der verstärkten Membran nach Anspruch 1 aufgebracht wird.

4. Verfahren gemäß Anspruch 3, bei welchem das Tintenmaterial in Streifen (12) aufgebracht wird.

5. Filmmaterial mit einem zusammenhängenden feuchtigkeitdampf-permeablen Teil (10), der zumindest eine partielle Tintenbeschichtung aufweist, die als ein Muster (12) auf einer Fläche des feuchtigkeit-dampf-permeablen Teils ausgebildet ist, um eine Verstärkung zum Versteifen des feuchtigkeitdampf-permeablen Teils (10) zu schaffen.

6. Filmmaterial gemäß Anspruch 5, bei welchem die Tinte eine nicht-giftige Tinte mit einem medizinisch zugelassenen Färbemittel aufweist

7. Filmmaterial gemäß Anspruch 6, bei welchem das Färbemittel ein Kupfer-Phthalocyanin-Färbemittel aufweist.

8. Filmmaterial gemäß Anspruch 5, bei welchem die Tinte in einem Träger oder Polyethylen gelöstes Styrol aufweist.

9. Filmmaterial gemäß Anspruch 8, bei welchem der Träger Methyl-Ethyl-Keton oder Alkohol aufweist.

10. Filmmaterial gemäß Anspruch 5, das ferner ein Klebemittel enthält, das auf dem feuchtigkeit-dampf-permeablen Teil aufgebracht ist.

## Revendications

1. Procédé pour renforcer une membrane (10) de polyurethane perméable à la vapeur d'humidité, comprenant l'application d'un revêtement de matériau formant encre suivant un motif (12) sur la membrane de polyurethane pour rendre la membrane (10) plus solide.

2. Procédé pour renforcer une membrane de polyurethane conforme à la revendication 1, dans lequel ledit matériau est appliqué en bandes (12).

3. Procédé pour réaliser une membrane de polyurethane renforcée perméable à la vapeur d'humidité revêtue d'un adhésif, comprenant l'application d'un adhésif sur la face inférieure de la membrane renforcée conforme à la revendication 1.

4. Procédé conforme à la revendication 3, dans lequel ledit matériau formant encre est appliqué en bandes (12).

5. Matériau en forme de film, comprenant un élément (10) perméable à la vapeur d'humidité comportant au moins un revêtement partiel d'encre formé suivant un motif (12) sur une surface dudit élément perméable à la vapeur d'humidité pour fournir un renforcement afin de rendre plus solide ledit élément (10) perméable à la vapeur d'humidité.

6. Matériau en forme de film conforme à la revendication 5, dans lequel ladite encre comprend une encre non toxique combinée à un colorant médicalement approuvé.

7. Matériau en forme de film conforme à la revendication 6, dans lequel ledit colorant comprend un colorant cuivre-phtalocyanine.

8. Matériau en forme de film conforme à la revendication 5, dans lequel ladite encre comprend du styrène dissous dans un support, ou du polyéthylène.

9. Matériau en forme de film conforme à la revendication 8, dans lequel le support comprend de la méthyle-éthyle-cétone ou de l'alcool.

10. Matériau en forme de film conforme à la revendication 5, comprenant en outre un adhésif appliqué sur ledit élément perméable à la vapeur d'humidité.
